# FASCICULE DE BREVET EUROPEEN

(11) **EP 2 388 042 B1**
(45) Date de publication et mention de la délivrance du brevet: **29.04.2020**
(21) Numéro de dépôt: 11166964.4
(22) Date de dépôt: 20.05.2011
(51) Int. Cl.: A61M 39/10, A61J 1/10, A61M 5/00, A61J 1/00, A61J 1/14

(54) **Connecteur pour un circuit de liquide**
Anschluss für einen Flüssigkeitskreislauf
Connector for a liquid circuit

(30) Priorité: 21.05.2010 FR 1053956
(43) Date de publication de la demande: 23.11.2011
(73) Titulaire: Technoflex, 64210 Bidart (FR)
(72) Inventeur: Curutcharry, Jean, F-64210 Guethary (FR); Viel, Frédéric, 64480 Ustaritz (FR); Capitaine, François, 64600 Anglet (FR)
(74) Mandataire: Regimbeau

(56) Documents cités:
- EP-A1- 2 161 013
- WO-A2-2006/042692
- WO-A2-2010/029371
- US-A- 4 349 024

## Description

La présente invention concerne un connecteur pour un circuit de liquide, notamment à usage médical.

Elle vise encore un circuit de liquide équipé d'au moins un tel connecteur pour l'administration à des fins médicales de substances liquides.

On connaît des connecteurs destinés à être introduits partiellement dans un moyen d'accès au contenu d'un récipient souple pour assurer la vidange de ce récipient.

Par exemple, la demande internationale WO2006/042692 divulgue un port permettant le remplissage ou la vidange d'un sac plastique pouvant contenir des solutions liquides. La demande internationale WO2010/029371 divulgue une cartouche destinée à être utilisée avec un récipient pour perfusion. La cartouche comprend un corps ayant une première extrémité et une deuxième extrémité et un passage entre celles-ci.

Ce moyen d'accès au contenu du récipient tel qu'une poche souple, prend généralement la forme d'une cheminée, laquelle est obtenue par l'insertion d'une portion de tube entre les deux parois de matière plastique souple constitutives du récipient et assemblage de l'ensemble ainsi disposé par soudure au moins des bords périphériques des parois.

Pour établir une communication de liquide entre l'intérieur du récipient et l'extérieur, un opérateur doit alors introduire manuellement l'extrémité du connecteur dans la cheminée.

Cette opération d'assemblage doit être simple pour l'opérateur afin de diminuer les risques d'erreur de manipulation. Cette condition a, d'ailleurs, conduit à la définition d'exigences relatives à la manipulation des récipients en plastique. On citera notamment la norme iso 15747 qui définit une limite maximale de la force nécessaire à un opérateur pour l'introduction d'un trocart dans une tubulure.

Or, on observe que cette opération est rendue difficile par la nature même des matériaux mis en œuvre dans le domaine médical.

En effet, ce connecteur et cette cheminée sont typiquement réalisés à base de polypropylène (PP). Cette matière thermoplastique tend à remplacer le chlorure de polyvinyle (PVC) dans la fabrication des éléments pour le stockage et le transfert de liquide à usage médical en raison de sa grande inertie vis-à-vis des solutions médicales.

Or, le contact de deux pièces réalisées à base de polypropylène ne favorise pas un déplacement relatif de celles-ci, et par conséquent, l'introduction du connecteur dans la cheminée est gênée.

Par ailleurs, cette cheminée peut comporter une membrane d'étanchéité interne pour garantir l'asepsie du contenu du récipient. Cette membrane qui présente une zone de renfort sur son pourtour afin d'empêcher une éventuelle introduction dans le circuit de liquide d'un morceau de celle-ci après déchirure, gène l'introduction de l'extrémité du connecteur dans la cheminée.

On a tenté de résoudre ces problèmes en réalisant un chanfrein à l'extrémité du connecteur pour faciliter son introduction dans la cheminée.

Si l'assemblage d'un connecteur présentant un tel chanfrein et d'un moyen d'accès peut être réalisé avec plus ou moins de facilités par un opérateur qualifié, l'extrémité de ce connecteur ne présente plus une rigidité suffisante pour autoriser un assemblage automatisé.

Or, la standardisation des dimensions des moyens d'accès aux récipients médicaux n'autorise pas, par exemple, à élargir leur diamètre pour faciliter un tel assemblage.

Par ailleurs, ces connecteurs sont généralement obtenus par injection d'une matière thermoplastique dans un moule, des broches étant mises en oeuvre pour délimiter le canal interne de circulation des liquides dans ces connecteurs.

Or, la nécessité d'assurer le retrait des broches empêche de jouer sur la géométrie de l'extrémité du connecteur lors de sa fabrication pour faciliter un assemblage automatisé.

La présente invention vise à pallier ces divers inconvénients en proposant un connecteur médical particulièrement simple dans sa conception et dans son mode opératoire, économique et permettant une introduction automatisée de celui-ci dans des moyens d'accès aux récipients connus de l'état de l'art.

A cet effet, l'invention concerne un connecteur pour un circuit de liquide comprenant une chambre tubulaire ou sensiblement tubulaire entre une première extrémité et une deuxième extrémité dudit connecteur, une première portion de ladite chambre délimitant un canal interne de diamètre d₁. Le connecteur est d'une seule pièce en matière thermoplastique, comporte au moins un élément d'étanchéité placé à ladite deuxième extrémité du connecteur ou respectivement dans ladite chambre, ledit au moins un élément d'étanchéité obturant ladite deuxième extrémité ou respectivement ladite chambre, ladite deuxième portion délimitant un canal interne de diamètre d₂ tel que d₁-d₂ > 0,1 d₁.

Selon l'invention,
- ladite première portion est placée entre ledit au moins un élément d'étanchéité et ladite première extrémité,
- ladite chambre comprend au moins une deuxième portion dite portion déformée, distincte de ladite première portion, ladite deuxième portion étant placée entre ladite première portion et la première extrémité dudit connecteur,
- ladite deuxième portion est placée dans une zone de ladite chambre délimitant un canal interne de diamètre décroissant en allant de ladite première portion vers ladite première extrémité,
et ladite deuxième portion présente une même épaisseur que ladite première portion.

Ce connecteur est particulièrement adapté pour établir une communication de liquide dans un circuit de liquide à usage médical.

On entend par le "connecteur étant d'une seule pièce" que ce connecteur est d'un seul tenant et ne résulte donc pas de l'assemblage de pièces initialement distinctes.

Ladite au moins deuxième portion dite déformée ne résultant pas de l'application d'un moyen externe tel qu'une bague de serrage par exemple, la surface externe dudit connecteur au droit de ces portions dites déformées est avantageusement libre.

Ladite au moins deuxième portion résulte, de préférence, d'une déformation du connecteur après fabrication de ce dernier. La surface externe dudit connecteur au droit de ladite au moins deuxième portion est par conséquent placée en retrait par rapport à la surface externe dudit connecteur au droit de ladite première portion. Toutefois, la paroi du connecteur au niveau des première et deuxième portions peut présenter une même épaisseur d'où une rigidité accrue par rapport aux connecteurs de l'état de l'art.

Il est à noter que le connecteur étant réalisé, à titre purement illustratif, par injection d'une matière thermoplastique, le positionnement de la deuxième portion dans le connecteur interdirait le retrait de la broche délimitant le canal interne au niveau de la première extrémité. En conséquence, cette deuxième portion n'est pas obtenue directement lors du moulage de la pièce.

Par contre, la chambre du connecteur peut comporter une ou plusieurs autres portions délimitant chacune un canal de diamètre supérieur au diamètre d₁ de ladite première portion.

Dans différents modes de réalisation particuliers de ce connecteur, chacun ayant ses avantages particuliers et susceptibles de nombreuses combinaisons techniques possibles:
- ledit au moins un élément d'étanchéité est sécable,
- ledit au moins un élément d'étanchéité est choisi dans le groupe comprenant une membrane, un bouchon, un bouchon quart de tour, un opercule et des combinaisons de ces éléments,
- ledit connecteur comporte sur au moins une partie de sa surface externe un ou plusieurs éléments choisis dans le groupe comprenant un jonc, un filetage, une rainure hélicoïdale, une collerette et un cran dont la face déterminant sa périphérie est inclinée.

De préférence, ce connecteur comporte sur sa surface externe une collerette ou bague formant une butée et dont la position par rapport à ladite première extrémité de ce connecteur permet de déterminer les dimensions de ladite deuxième portion pour un outil de déformation donné.
- le diamètre d₂ de ladite deuxième portion dite déformée est tel que d₁-d₂ ≥ 0,1 d₁ quelle que soit la température Tc dudit connecteur avec Tc comprise entre [T_{RT}, T_{entrée}[ où T_{RT} est la température ambiante et T_{entrée} est la température d'entrée en fusion dudit matériau thermoplastique.

La température d'entrée en fusion est encore appelée température d'onset de fusion.

A titre purement illustratif, le connecteur étant réalisé à base de polypropylène, la température de fusion de cette matière est de l'ordre de 142°C et sa température d'entrée en fusion est de l'ordre de 130°C. En restant en deçà de 130°C, on observe que le diamètre d₂ de ladite deuxième portion dite déformée est tel que d₁-d₂ ≥ 0,1 d₁.
- ladite au moins deuxième portion est placée à ladite première extrémité dudit connecteur pour faciliter l'assemblage dudit connecteur avec un conduit de diamètre interne D supérieur au diamètre externe dudit connecteur au droit de ladite deuxième portion,
- ladite deuxième portion délimite un canal interne de diamètre d₂ tel que d₁-d₂ ≥ 0,15 d₁, et encore mieux 0,2 d₁.

L'invention vise également un circuit de liquide comprenant un moyen d'accès au contenu d'un récipient tel qu'une poche souple, un connecteur tel que décrit précédemment étant inséré au moins partiellement dans ledit moyen d'accès pour établir une communication de liquide.

A titre illustratif, dans le cas d'un circuit de liquide pour perfusion, ce circuit comprend un récipient souple comportant un volume intérieur, au moins un moyen d'accès tel qu'une cheminée, au volume intérieur de ce récipient et un connecteur tel que décrit précédemment.

Ce connecteur peut, de plus, être relié à un conduit flexible d'acheminement du contenu du récipient souple en vue d'une injection par voie intraveineuse par exemple.

L'invention sera décrite plus en détail en référence aux dessins annexés dans lesquels:
- la figure 1 est une représentation schématique d'un connecteur médical selon un premier mode de réalisation de l'invention ;
- la figure 2 est une vue en coupe du connecteur de la Figure 1 ;
- la figure 3 est une vue en perspective d'un connecteur médical selon un deuxième mode de réalisation de l'invention ;
- la figure 4 est une vue en coupe d'un connecteur médical selon un troisième mode de réalisation de l'invention ;

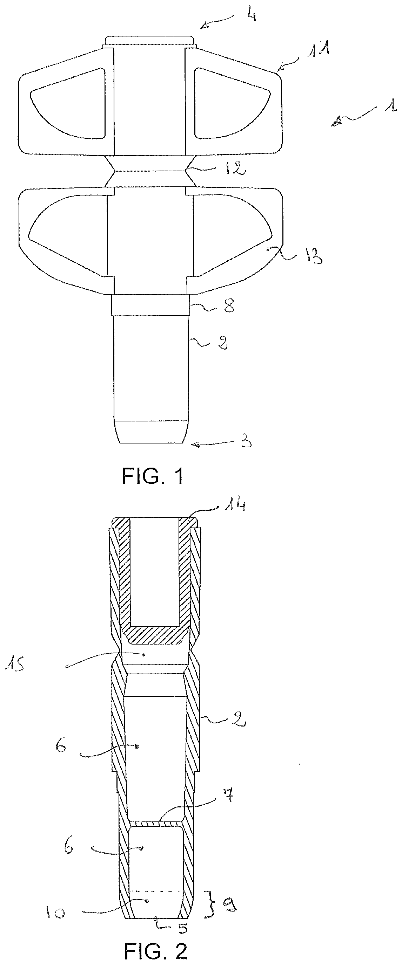

Les Figures 1 et 2 montrent un connecteur médical selon un mode de réalisation préféré de l'invention.

Le connecteur est d'un seul tenant et obtenu ici par moulage par injection d'une matière thermoplastique médicalement inerte. Il peut, à titre purement illustratif, être réalisé en polycarbonate ou des dérivés de celui-ci, en polyoléfine (tel que le polyéthylène, le polypropylène, un homopolymère ou un copolymère de polypropylène) ou en polystyrène, ou encore en polyoléfine comprenant un thermoplastique élastomère (TPE) tel que le SEB (copolymère bloc Styrène, Ethylène, Butylène), le SEBS (copolymère bloc Styrène, Ethylène, Butylène, Styrène)...

Ce connecteur 1 comporte une tubulure 2 définissant intérieurement un canal d'écoulement des liquides entre une extrémité amont 3 et une extrémité aval 4 de ce connecteur, ces extrémités 3, 4 étant considérées dans le sens d'écoulement des liquides depuis une poche souple auquel ce connecteur 1 serait solidaire. L'extrémité amont 3 comprend un orifice d'entrée 5 des liquides. Ce canal est tout d'abord délimité par une première portion 6 tubulaire ou sensiblement tubulaire de diamètre d₁.

Cette première portion 6 est séparée par une membrane d'étanchéité 7 interne. Cette membrane d'étanchéité 7 qui est sécable, obture le passage pour le fluide destiné à passer dans le connecteur.

La tubulure 2 comporte également sur sa surface externe une collerette 8, ou bague, qui assure, d'une part, la prise en main ou à l'aide d'un robot de ce connecteur 1 et, d'autre part, forme une butée dont la position par rapport à l'extrémité amont 3 de ce connecteur a permis de déterminer lors d'une étape de mise en forme de ce connecteur sur un outil de déformation, les dimensions de sa partie dite déformée 9.

Cette partie dite déformée 9, ou encore plissée, définit ainsi une portion de canal 10 distinct de celui délimité par la première portion tubulaire 6 et placé entre la membrane d'étanchéité 7 et l'extrémité amont 3 du connecteur. Cette portion de canal 10 présente une forme rétrécie, c'est-à-dire qu'il a un diamètre d₂ qui va décroissant en allant vers l'extrémité amont 3 du connecteur.

Ce diamètre d₂ vérifie cependant en tout point la relation suivante d₁-d₂ ≥ 0,1 d₁ quelque soit la température Tc de ce connecteur avec Tc comprise entre [T_{RT}, T_{entrée}[ où T_{RT} est la température ambiante et T_{entrée} est la température d'entrée en fusion dudit matériau thermoplastique.

Un élément sécable 11 est également placé à l'extrémité aval 4 du connecteur en étant relié par un mince pontage 12 à l'extrémité supérieure de la tubulure 2. Cet élément sécable 11 est un bouchon quart de tour. Ce bouchon quart de tour 11 peut être désolidarisé de la tubulure 2 par une simple rotation manuelle d'un quart de tour. La tubulure 2 comporte également sur sa surface externe des ailettes 13 de préhension permettant de séparer aisément le bouchon quart de tour 11 du corps de la tubulure 2.

Ce bouchon quart de tour 11 comporte dans sa partie supérieure un bouchon 14 obturant de manière hermétique un canal 15 de ce bouchon qui est en communication de fluide avec le canal délimité par la première portion tubulaire 6 de la tubulure 2.

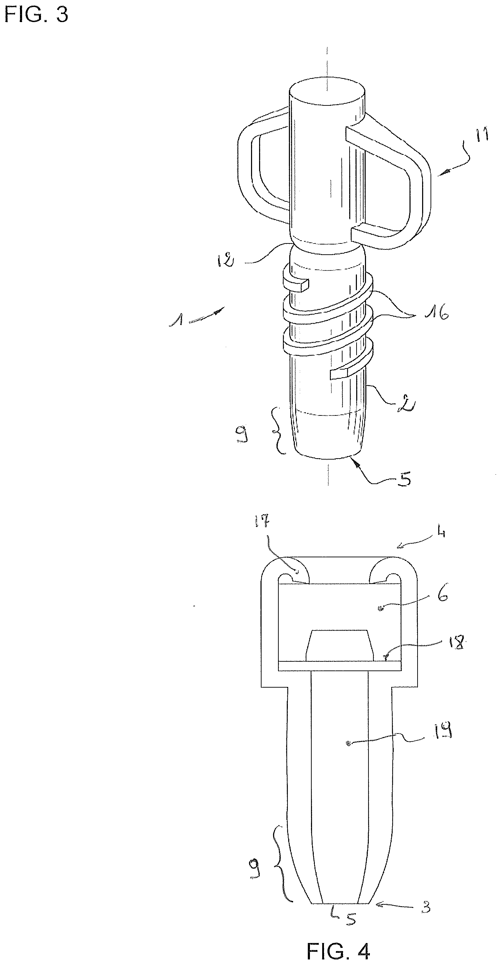

La Figure 3 montre un connecteur selon un deuxième mode de réalisation de l'invention. Les éléments de la Figure 3 portant les mêmes références que les éléments des Figures 1 et 2 représentent les mêmes objets, lesquels ne seront pas décrits de nouveau ci-dessous.

Ce connecteur 1 se distingue de celui décrit aux Figures 1 et 2, notamment en ce qu'il comporte une tubulure 2 présentant sur sa surface externe un filetage 16 destiné à coopérer, de manière connue, avec une virole cylindrique d'accouplement d'un connecteur luer mâle d'un set de transfert par exemple.

Par ailleurs, ce connecteur ne comporte pas d'ailettes de préhension 13.

A titre purement illustratif, et dans un mode de mise en œuvre particulier de la présente invention, un connecteur comportant une tubulure 2 droite de diamètre constant d₁ égal à 7,10 mm et de longueur 11,7 mm, a vu son extrémité amont 3 plissée, ou déformée, sur un outil de déformation dont l'angle de sertissage est de 45°. La distance séparant l'extrémité inférieure de la collerette 8 de l'extrémité amont 3 de ce connecteur avant déformation est de 10,45 mm. Le diamètre d₂ observé de l'orifice d'entrée 5 de l'extrémité amont 3 est de 5,10 +/- 0,20 mm, soit d₁-d₂= 2 +/- 0,20 mm > 0,1 d₁. L'angle de sertissage est de préférence compris entre 15° et 75°, et encore mieux entre 30° et 45° par rapport à l'axe longitudinal de la tubulure.

La Figure 4 est une vue en coupe d'un connecteur médical selon un troisième mode de réalisation de l'invention. Les éléments de la Figure 4 portant les mêmes références que les éléments des Figures 1 et 2 représentent les mêmes objets, lesquels ne seront pas décrits de nouveau ci-dessous. Ce connecteur comporte un canal interne pour le passage d'un fluide.

Une première partie de ce canal est délimitée par une première portion 6 tubulaire du connecteur en présentant un diamètre d₁. Cette première portion 6 comporte à son extrémité déterminant l'extrémité aval 4 du connecteur un bourrelet d'étanchéité 17. Elle est par ailleurs séparée par un obturateur étanche et sécable 18 d'une deuxième portion tubulaire 19 définissant un canal de diamètre interne constant d₂ restreint par rapport au diamètre d₁ du canal délimité par la première portion 6.

Cette deuxième portion 19 est prolongée en allant vers l'extrémité amont 3 du connecteur par une troisième portion 9 dite déformée. Cette portion 9 dite déformée est distincte de la première portion 6 en étant placée entre l'obturateur étanche 18 et l'extrémité amont 3 du connecteur.

Cette portion dite déformée 9 délimite un canal interne de diamètre décroissant en allant de la deuxième portion 18 du connecteur vers l'extrémité amont 5 de celui-ci.

Cette portion 9 dite déformée comporte un ensemble de n parties déformées successives dont au moins une partie délimitant une portion de canal interne de diamètre d₃ est telle que d₁-d₃ ≥ 0,10 d₁ quelque soit la température Tc de ce connecteur avec Tc comprise entre [T_{RT}, T_{entrée}[ où T_{RT} est la température ambiante et T_{entrée} est la température d'entrée en fusion dudit matériau thermoplastique.

Puisque cette partie est placée dans une zone du canal interne du connecteur de diamètre décroissant, on peut définir j autres parties du connecteur vérifiant d₁-dj > 0,10 d₁ avec j supérieur ou égal à 3. L'orifice d'entrée 5 présente ici un diamètre d_{orifice} tel que d₁-d_{orifice} ≥ 0,2 d₁.

On observe que la surface externe du connecteur au niveau, i.e. au droit, de ces parties déformées est placée en retrait par rapport à la surface externe du connecteur au niveau de la deuxième portion 19.

## Revendications

1. Connecteur pour un circuit de liquide comprenant une chambre tubulaire ou sensiblement tubulaire entre une première extrémité (3) et une deuxième extrémité (4) dudit connecteur, une première portion (6) de ladite chambre délimitant un canal interne de diamètre d₁, ledit connecteur étant d'une seule pièce en matière thermoplastique, ledit connecteur comportant au moins un élément d'étanchéité (7, 11, 18) placé à ladite deuxième extrémité (4) du connecteur ou respectivement dans ladite chambre, ledit au moins un élément d'étanchéité (7, 11, 18) obturant ladite deuxième extrémité (4) ou respectivement ladite chambre, ladite deuxième portion (9) délimitant un canal interne de diamètre d₂ tel que d₁-d₂ ≥ 0,1 d₁ et ledit connecteur étant **caractérisé en ce que** :
- ladite première portion (6) est placée entre ledit au moins un élément d'étanchéité (7, 11, 18) et ladite première extrémité (3),
- ladite chambre comprend au moins une deuxième portion (9) dite portion déformée, distincte de ladite première portion (6), ladite deuxième portion (9) étant placée entre ladite première portion (6) et la première extrémité (3) dudit connecteur,
- ladite deuxième portion (9) est placée dans une zone de ladite chambre délimitant un canal interne de diamètre décroissant en allant de ladite première portion (6) vers ladite première extrémité (3),
et ladite deuxième portion (9) présente une même épaisseur que ladite première portion.

2. Connecteur selon la revendication 1, **caractérisé en ce que** ledit au moins un élément d'étanchéité (7) est sécable.

3. Connecteur selon la revendication 1 ou 2, **caractérisé en ce que** ledit au moins un élément d'étanchéité (7, 11, 18) est choisi dans le groupe comprenant une membrane, un bouchon, un bouchon quart de tour, un opercule et des combinaisons de ces éléments.

4. Connecteur selon l'une quelconque des revendications 1 à 3, **caractérisé en ce que** ledit connecteur comporte sur au moins une partie de sa surface externe un ou plusieurs éléments (13, 16) choisis dans le groupe comprenant un jonc, un filetage, une rainure hélicoïdale, une collerette et un cran dont la face déterminant sa périphérie est inclinée.

5. Connecteur selon la revendication 4, **caractérisé en ce que** ledit connecteur comporte sur sa surface externe une collerette (13) ou bague formant une butée et dont la position par rapport à ladite première extrémité (3) dudit connecteur permet de déterminer les dimensions de ladite deuxième portion (9) pour un outil de déformation donné.

6. Connecteur selon l'une quelconque des revendications 1 à 5, **caractérisé en ce que** le diamètre d₂ de ladite deuxième portion (9) dite déformée est tel que d₁-d₂ ≥ 0,1 d₁ quelle que soit la température Tc dudit connecteur avec Tc comprise entre [T_{RT}, T_{entrée}[ où T_{RT} est la température ambiante et T_{entrée} est la température d'entrée en fusion dudit matériau thermoplastique.

7. Connecteur selon l'une quelconque des revendications 1 à 6, **caractérisé en ce que** ladite deuxième portion (9) est placée à ladite première extrémité (3) dudit connecteur pour faciliter l'assemblage dudit connecteur avec un conduit de diamètre interne D supérieur au diamètre externe dudit connecteur au droit de ladite deuxième portion (9).

## Patentansprüche

1. Anschluss für einen Flüssigkeitskreislauf, umfassend eine rohrförmige oder etwa rohrförmige Kammer zwischen einem ersten Ende (3) und einem zweiten Ende (4) des Anschlusses, wobei ein erster Abschnitt (6) der Kammer einen inneren Kanal mit dem Durchmesser d₁ begrenzt, wobei der Anschluss ein einziges Teil aus thermoplastischem Material ist, wobei der Anschluss mindestens ein Dichtungselement (7, 11, 18) aufweist, das am zweiten Ende (4) des Anschlusses oder beziehungsweise in der Kammer platziert ist, wobei das mindestens eine Dichtungselement (7, 11, 18) das zweite Ende (4) oder beziehungsweise die Kammer abdichtet, wobei der zweite Abschnitt (9) einen inneren Kanal mit dem Durchmesser d₂ begrenzt, so dass d₁-d₂ ≥ 0,1 d₁ ist und der Anschluss **dadurch gekennzeichnet ist, dass**:
- der erste Abschnitt (6) zwischen dem mindestens einen Dichtungselement (7, 11, 18) und dem ersten Ende (3) platziert ist,
- die Kammer mindestens einen zweiten, als verformter Abschnitt bezeichneten Abschnitt (9) umfasst, der sich von dem ersten Abschnitt (6) unterscheidet, wobei der zweite Abschnitt (9) zwischen dem ersten Abschnitt (6) und dem ersten Ende (3) des Anschlusses platziert ist,
- wobei der zweite Abschnitt (9) in einer Zone der Kammer platziert ist, die einen inneren Kanal mit einem vom ersten Abschnitt (6) zum ersten Ende (3) abnehmenden Durchmesser begrenzt,
und der zweite Abschnitt (9) dieselbe Stärke wie der erste Abschnitt aufweist.

2. Anschluss nach Anspruch 1, **dadurch gekennzeichnet, dass** das mindestens eine Dichtungselement (7) trennbar ist.

3. Anschluss nach Anspruch 1 oder 2, **dadurch gekennzeichnet, dass** das mindestens eine Dichtungselement (7, 11, 18) aus der Gruppe ausgewählt ist, die eine Membran, einen Stopfen, einen Vierteldrehungsstopfen, eine Kappe und Kombinationen dieser Elemente umfasst.

4. Anschluss nach einem der Ansprüche 1 bis 3, **dadurch gekennzeichnet, dass** der Anschluss auf mindestens einem Teil seiner äußeren Oberfläche ein oder mehrere Elemente (13, 16) aufweist, die aus der Gruppe ausgewählt sind, die einen Stab, ein Gewinde, eine schraubenförmige Rille, einen Kragen und eine Raste umfasst, deren Seite, die ihre Peripherie bestimmt, geneigt ist.

5. Anschluss nach Anspruch 4, **dadurch gekennzeichnet, dass** der Anschluss auf seiner äußeren Oberfläche einen Kragen (13) oder Ring aufweist, der einen Anschlag bildet und dessen Position in Bezug auf das erste Ende (3) des Anschlusses erlaubt, die Abmessungen des zweiten Abschnitts (9) für ein bestimmtes Verformungswerkzeug zu bestimmen.

6. Anschluss nach einem der Ansprüche 1 bis 5, **dadurch gekennzeichnet, dass** der Durchmesser d₂ des als verformt bezeichneten zweiten Abschnitts (9) derart ist, dass d₁-d₂ ≥ 0,1 d₁ ist, unabhängig von der Temperatur Tc des Anschlusses mit Tc zwischen [T_{RT}, T_{Beginn}[, wobei T_{RT} die Raumtemperatur ist und T_{Beginn} die Temperatur des Beginns der Schmelze des thermoplastischen Materials ist.

7. Anschluss nach einem der Ansprüche 1 bis 6, **dadurch gekennzeichnet, dass** der zweite Abschnitt (9) am ersten Ende (3) des Anschlusses platziert ist, um die Verbindung des Anschlusses mit einer Leitung mit einem Innendurchmesser D größer als der Außendurchmesser des Anschlusses senkrecht zum zweiten Abschnitt (9) zu erleichtern.

## Claims

1. A connector for a liquid circuit comprising a tubular or substantially tubular chamber between a first end (3) and a second end (4) of said connector, a first portion (6) of said chamber delimiting an internal channel of diameter d₁, said connector being in a single piece made of thermoplastic material, said connector including at least one sealing element (7, 11, 18) placed at said second end (4) of the connector or respectively in said chamber, said at least one sealing element (7, 11, 18) closing off said second end (4) or respectively said chamber, said second portion (9) delimiting an internal channel of diameter d₂ such that d₁-d₂ ≥ 0.1 d₁ and said connector being **characterized in that**:
- said first portion (6) is placed between said at least one sealing element (7, 11, 18) and said first end (3),
- said chamber comprises at least a second portion (9) called deformed portion, distinct from said first portion (6), said second portion (9) being placed between said first portion (6) and the first end (3) of said connector,
- said second portion (9) is placed in an area of said chamber delimiting an internal channel of decreasing diameter going from said first portion (6) to said first end (3),
and said second portion (9) has the same thickness as said first portion.

2. The connector according to claim 1, **characterized in that** said at least one sealing element (7) is divisible.

3. The connector according to claim 1 or 2, **characterized in that** said at least one sealing element (7, 11, 18) is chosen from the group comprising a membrane, a stopper, a twist-off stopper, a cap and combinations of these elements.

4. The connector according to any one of claims 1 to 3, **characterized in that** said connector includes on at least part of its outer surface one or more elements (13, 16) chosen from the group comprising a bead, a thread, a helical groove, a flange and a notch whose face determining its periphery is inclined.

5. The connector according to claim 4, **characterized in that** said connector includes on its outer surface a flange (13) or ring forming an abutment and whose position relative to said first end (3) of said connector makes it possible to determine the dimensions of said second portion (9) for a given deformation tool.

6. The connector according to any one of claims 1 to 5, **characterized in that** the diameter d₂ of said second portion (9) called deformed portion is such that d₁-d₂ ≥ 0.1 d₁ whatever the temperature Tc of said connector, Tc ranging between [T_{RT}, T_{INPUT}[ where T_{RT} is the ambient temperature and T_{INPUT} is the meltdown input temperature of said thermoplastic material.

7. The connector according to any one of claims 1 to 6, **characterized in that** said second portion (9) is placed at said first end (3) of said connector to facilitate the assembly of said connector with a duct of internal diameter D greater than the external diameter of said connector in line with said second portion (9).
